# EUROPÄISCHE PATENTSCHRIFT

(11) **EP 1 628 940 B1**
(45) Veröffentlichungstag und Bekanntmachung des Hinweises auf die Patenterteilung: **23.04.2008**
(21) Anmeldenummer: 04733803.3
(22) Anmeldetag: 19.05.2004
(51) Int. Cl.: C07C 7/08, C07C 11/167

(54) **VERFAHREN ZUR GEWINNUNG VON ROH-1,3-BUTADIEN AUS EINEM C4-SCHNITT**
METHOD FOR OBTAINING CRUDE 1,3-BUTADIENE FROM A C4 FRACTION
PROCEDE D'EXTRACTION DE 1,3-BUTADIENE BRUT A PARTIR D'UNE FRACTION DE C4

(30) Priorität: 20.05.2003 DE 10322655
(43) Veröffentlichungstag der Anmeldung: 01.03.2006
(73) Patentinhaber: BASF SE, 67056 Ludwigshafen (DE)
(72) Erfinder: HEIDA, Bernd, 67158 Ellerstadt (DE)
(74) Vertreter: Isenbruck, Günter
(86) Internationale Anmeldenummer: PCT/EP2004/005407
(87) Internationale Veröffentlichungsnummer: WO 2004/103937

(56) Entgegenhaltungen:
- DE-A- 10 102 168
- DE-A- 10 105 660

## Beschreibung

Die Erfindung betrifft ein Verfahren zur Gewinnung von Roh-1,3-Butadien durch Extraktivdestillation aus einem C₄-Schnitt mit einem selektiven Lösungsmittel.

Die Gewinnung von Roh-1,3-Butadien aus einem C₄-Schnitt ist wegen der geringen Unterschiede der Komponenten des C₄-Schnittes in den relativen Flüchtigkeiten ein kompliziertes destillationstechnisches Problem. Daher wird die Auftrennung durch eine sogenannte Extraktivdestillation durchgeführt, das heißt eine Destillation unter Zugabe eines Extraktionsmittels, das einen höheren Siedepunkt als das aufzutrennende Gemisch aufweist und das die Unterschiede in den relativen Flüchtigkeiten der aufzutrennenden Komponenten erhöht. Durch Einsatz geeigneter Extraktionsmittel kann aus dem genannten C₄-Schnitt mittels Extraktivdestillation eine Roh-1,3-Butadienfraktion erhalten werden, die anschließend in Reindestillationskolonnen weitergereinigt wird neben einem Strom, der die weniger löslichen Kohlenwasserstoffe als 1,3-Butadien, insbesondere Butane und Butene enthält, sowie einem Strom, der die leichter löslichen Kohlenwasserstoffe als 1,3-Butadien, insbesondere die Butine sowie gegebenenfalls 1,2-Butadien enthält.

Vorliegend wird als Roh-1,3-Butadien ein Kohlenwasserstoffgemisch bezeichnet, dass aus einem C₄-Schnitt gewonnen wurde, aus dem mindestens 90 Gew.-% der Summe aus Butanen und Butenen, bevorzugt mindestens 96 Gew.-% der Summe aus Butanen und Butenen, besonders bevorzugt mindestens 99 Gew.-% der Summe aus Butanen und Butenen und gleichzeitig mindestens 90 Gew.-% der C₄-Acetylene, bevorzugt mindestens 96 Gew.-% der C₄-Acetylene, besonders bevorzugt mindestens 99 Gew.-% der C₄-Acetylene, abgetrennt wurden. Roh-1,3-Butadien enthält das Wertprodukt 1,3-Butadien häufig in einem Anteil von mindestens 80 Gew.-%, bevorzugt 90 Gew.-%, besonders bevorzugt 95 Gew.-%, Rest Verunreinigungen.

Dem gegenüber wird als Rein-1,3-Butadien ein Kohlenwasserstoffgemisch bezeichnet, das das Wertprodukt 1,3-Butadien in einem Anteil von mindestens 99 Gew.-%, bevorzugt 99,5 Gew.-%, besonders bevorzugt 99,7 Gew.-%, Rest Verunreinigungen, enthält.

Aus DE 101 05 660.5 ist ein Verfahren mit vereinfachter konstruktiver Ausgestaltung der Apparate gegenüber älteren Verfahren bekannt: die Auftrennung des C₄-Schnitts erfolgt in einer Trennwandkolonne mit bis zum oberen Ende der Trennwandkolonne durchgezogener Trennwand und einer der Trennwandkolonne vorgeschalteten Extraktivwaschkolonne.

Der Offenbarungsgehalt der vorgenannten DE 101 05 660.5 wird hiermit voll umfänglich in die vorliegende Patentanmeldung einbezogen.

Nach dem Verfahren der DE 101 05 660.5 wird aus dem Sumpf der zur Extraktivdestillation eingesetzten Trennwandkolonne ein teilentgaster Lösungsmittelstrom abgezogen. Der Begriff "teilentgastes Lösungsmittel" ist hierbei dem in der Extraktivdestillation zur Gewinnung von 1,3-Butadien tätigen Fachmann bekannt und bezeichnet ein selektives Lösungsmittel, das noch gelöste Komponenten aus dem aufzutrennenden C₄-Schnitt enthält und zwar die Komponenten, die die größte Affinität zum selektiven Lösungsmittel aufweisen. Hierzu zählen insbesondere die C₄-Acetylene, vor allem Ethylacetylen und Vinylacetylen.

Ein lediglich teilentgaster Lösungsmittelstrom kann jedoch nicht in die Extraktivdestillation recycliert werden, da sich ansonsten die spezifikationsschädlichen Acetylene aufpegeln würden. Daher musste der aus der Trennwandkolonne abgezogene Sumpfstrom vor der Recyclierung in die Extraktivdestillation zunächst einer Ausgaserkolonne, wie sie zum Beispiel aus DE-A 27 24 365 bekannt ist, zugeführt werden, die bei niedrigerem Druck gegenüber der Kolonne, aus deren Sumpf der teilentgaste Strom abgezogen wird, betrieben wird. In der Ausgaserkolonne erfolgt die Aufbereitung des teilentgasten Lösungsmittelstromes unter Erhalt eines gereinigten, das heißt vollständig entgasten Lösungsmittels am Sumpf und eines gasförmigen Kohlenwasserstromes am Kopf der Ausgaserkolonne, der über einen Kompressor in den unteren Bereich der Extraktivdestillationskolonne zurückgeführt wird.

Unter dem Begriff gereinigtes Lösungsmittel oder voll entgastes Lösungsmittel wird vorliegend ein Lösungsmittel verstanden, das so weit an Komponenten aus dem C₄-Schnitt abgereichert ist, dass es für den Einsatz als selektives Lösungsmittel zur Extraktivdestillation eines C₄-Schnitts geeignet ist, wobei die vorgegebenen Spezifikationen für Roh-1,3-Butadien und Raffinat 1 eingehalten werden. Schlüsselkomponenten sind hierbei C₄-Acetylene, insbesondere Ethylacetylen und Vinylacetylen.

Es war Aufgabe der Erfindung, ein wirtschaftlicheres Verfahren zur Gewinnung von Roh-1,3-Butadien durch Extraktivdestillation aus einem C₄-Schnitt zur Verfügung zu stellen, unter Erhalt von gereinigtem Lösungsmittel unmittelbar aus dem Sumpf der Extraktivdestillationskolonne.

Die Lösung geht aus von einem Verfahren zur Gewinnung von Roh-1,3-Butadien durch Extraktivdestillation aus einem C₄-Schnitt mit einem selektiven Lösungsmittel in einer Trennwandkolonne (TK) in der eine Trennwand (T) in Kolonnenlängsrichtung unter Ausbildung eines ersten Teilbereichs (A), eines zweiten Teilbereichs (B) und eines unteren gemeinsamen Kolonnenbereichs (C) angeordnet und der eine Extraktivwaschkolonne (K) vorgeschaltet ist.

Die Erfindung ist dadurch gekennzeichnet, dass man durch Regelung des Energieeintrags in die Trennwandkolonne (TK) über einen Sumpfverdampfer (V) und Auslegung der Zahl der theoretischen Trennstufen im unteren gemeinsamen Kolonnenbereich (C) die Fahrweise der Trennwandkolonne (TK) so einstellt, dass man aus der Trennwandkolonne (TK) einen Sumpfstrom (17) erhält, der aus gereinigtem Lösungsmittel besteht.

Es wurde überraschend gefunden, dass man gereinigtes Lösungsmittel, das in die Extraktivdestillation recycliert werden kann, unmittelbar aus dem Sumpf der Extraktivdestillationskolonne abziehen kann, ohne dass hierfür eine zusätzliche Ausgaserkolonne erforderlich wäre. Es ist hierfür möglich und ausreichend, den Energieeintrag in die Extraktivdestillationskolonne über den Sumpfverdampfer und die Anzahl der theoretischen Trennstufen im unteren gemeinsamen Kolonnenbereich der als Trennwandkolonne ausgebildeten Extraktivdestillationskolonne die Betriebsbedingungen so festzulegen, dass im Sumpf der Extraktivdestillationskolonne gereinigtes Lösungsmittel abgezogen werden kann.

Der vorliegend als Ausgangsgemisch einzusetzende sogenannte C₄-Schnitt ist ein Gemisch von Kohlenwasserstoffen mit überwiegend vier Kohlenstoffatomen pro Molekül. C₄-Schnitte werden beispielsweise bei der Herstellung von Ethylen und/oder Propylen durch thermisches Spalten einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an C₃- und C₅-Kohlenwasserstoffen, sowie Butine, insbesondere 1-Butin (Ethylacetylen) und Butenin(Vinylacetylen). Dabei beträgt der 1,3-Butadiengehalt im allgemeinen 10 bis 80 Ges.-%, vorzugsweise 20 bis 70 Gew.-%, insbesondere 30 bis 60 Gew.-%, während der Gehalt an Vinylacetylen und Ethylacetylen im allgemeinen 5 Gew.-% nicht übersteigt.

Für die eingangs bereits definierte Extraktivdestillation kommen für das vorliegende Trennproblem, der Gewinnung von 1,3-Butadien aus dem C₄-Schnitt, als Extraktionsmittel, d.h. als selektive Lösungsmittel, generell Substanzen oder Gemische in Frage, die einen höheren Siedepunkt als das aufzutrennende Gemisch sowie eine größere Affinität zu konjugierten Doppelbindungen und Dreifachbindungen als zu einfachen Doppelbindungen sowie Einfachbindungen aufweisen, bevorzugt dipolare, besonders bevorzugt dipolaraprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropioninitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden N-alkylsubstituierte niedere aliphatische Säureamide oder N-alkylsubstituierte cyclische Säureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, vorliegend abgekürzt als NMP bezeichnet, bevorzugt in wäßriger Lösung, insbesondere mit 7 bis 10 Gew.-% Wasser, besonders bevorzugt mit 8,3 Gew.-% Wasser.

Die Extraktivdestillation wird in einer Trennwandkolonne durchgeführt, in der eine Trennwand in Kolonnenlängsrichtung und Ausbildung eines ersten Teilbereichs, eines zweiten Teilbereichs und eines unteren gemeinsamen Kolonnenbereichs angeordnet ist und die mit einer vorgeschalteten Extraktivwaschkolonne verbunden ist.

Trennwandkolonnen werden in bekannter Weise für komplexere Trennaufgaben, in der Regel für Gemische von mindestens drei Komponenten, wobei die Einzelkomponenten jeweils in reiner Form erhalten werden sollen, eingesetzt. Sie weisen eine Trennwand auf, d.h. in der Regel ein in Kolonnenlängsrichtung ausgerichtetes ebenes Blech, das eine Quervermischung der Flüssigkeits- und Brüdenströme in Teilbereichen der Kolonne verhindert.

Vorliegend wird eine besonders ausgestaltete Trennwandkolonne eingesetzt, deren Trennwand bis am obersten Punkt der Kolonne durchgezogen ist und somit eine Vermischung von Flüssigkeits- und Brüdenströmen lediglich im unteren gemeinsamen Kolonnenbereich gestattet. Der sogenannte erste und zweite Teilbereich sind durch die Trennwand voneinander getrennt.

Die Extraktivwaschkolonne ist eine Gegenstromwaschkolonne.

Nach einer bevorzugten Verfahrensführung wird
- der C₄-Schnitt dem ersten Teilbereich der Trennwandkolonne, bevorzugt in dessen mittleren Bereich, zugeführt,
- der Kopfstrom aus dem ersten Teilbereich der Trennwandkolonne der Extraktivwaschkolonne, in deren unteren Bereich, zugeführt,
- in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
- die im selektiven Lösungsmittel weniger als 1,3-Butadien löslichen Komponenten des C₄-Schnittes werden über Kopf der Extraktivwaschkolonne abgezogen,
- der Sumpfstrom aus der Extraktivwaschkolonne in den oberen Bereich des ersten Teilbereichs der Trennwandkolonne zurückgeführt,
- der Trennwandkolonne im oberen Bereich des zweiten Teilbereichs ein zweiter Teilstrom des selektiven Lösungsmittels zugeführt,
- das Kopfprodukt aus dem zweiten Teilbereich (B) der Trennwandkolonne als Roh-1,3-Butadien abgezogen und
- aus dem unteren gemeinsamen Kolonnenbereich der Trennwandkolonne ein Sumpfstrom bestehend aus gereinigtem Lösungsmittel abgezogen wird, der in das Verfahren recycliert wird.

Bevorzugt wird somit der aufzutrennende C₄-Schnitt dem ersten Teilbereich der Trennwandkolonne, besonders bevorzugt in dessen mittleren Bereich, zugeführt;
der Kopfstrom aus dem ersten Teilbereich der Trennwandkolonne wird der vorgeschalteten Extraktivwaschkolonne in deren unteren Bereich zugeführt,
in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
die im selektiven Lösungsmittel weniger als 1,3-Butadien-löslichen Komponenten des C₄-Schnittes über Kopf der Extraktivwaschkolonne abgezogen, besonders bevorzugt in einem Kondensator am Kopf der Extraktivwaschkolonne kondensiert und teilweise als Rücklauf erneut auf die Extraktivwaschkolonne aufgegeben, im übrigen als ein überwiegend Butane und Butene enthaltendes Nebenprodukt, häufig als Raffinat 1 bezeichnet, abgezogen.

Durch die Zuführung des Sumpfstroms der Extraktivwaschkolonne, d.h. einem Strom, der neben dem selektiven Lösungsmittel 1,3-Butadien, Butane, Butene sowie die besser als 1,3-Butadien im selektiven Lösungsmittel löslichen Komponenten des C₄-Schnitts enthält, in den oberen Bereich des ersten Teilbereichs der Trennwandkolonne kann durch den Stoffaustausch zwischen diesem Strom und dem dampfförmig aufgegebenen C₄-Schnitt im oberen Bereich des ersten Teilbereichs der Trennwandkolonne eine Gegenstromextraktion unter Ausschleusung der im selektiven Lösungsmitttel weniger als das 1,3-Butadien löslichen Komponenten am Kopf des ersten Teilbereichs der Trennwandkolonne erfolgen.

Am unteren Ende der Trennwand fällt ein dampfförmiger Strom an, der neben 1,3-Butadien die im selektiven Lösungsmittel besser als 1,3-Butadien-löslichen Komponenten des C₄-Schnitts, insbesondere C₄-Acetylene, enthält. Diese werden aus dem aufsteigenden dampfförmigen Strom im Gegenstrom mit einem zweiten Teilstrom des selektiven Lösungsmittels, das im oberen Bereich des zweiten Teilbereichs der Trennwandkolonne aufgegeben wird, ausgewaschen. Das dampfförmige Kopfprodukt aus dem zweiten Teilbereich der Trennwandkolonne wird abgezogen, wobei es bevorzugt in einem Kondensator am Kolonnenkopf kondensiert, ein Teilstrom des kondensierten Kopfstromes zurück auf den Teilbereich B der Trennwandkolonne als Rücklauf gegeben und der kondensierte Kopfstrom wird im übrigen als Roh-1,3-Butadien abgezogen wird.

Im unteren gemeinsamen Kolonnenbereich erfolgt eine vollständige Entgasung des Lösungsmittels, wobei am Sumpf der Extraktivdestillationskolonne ein gereinigtes Lösungsmittel erhalten wird.

Bei der Bestimmung des hierfür erforderlichen Energieeintrags über den Sumpfverdampfer der Extraktivdestillationskolonne wird der Verfahrenstechniker die thermische Belastbarkeit der Substanz oder des Substanzgemisches berücksichtigen, die jeweils im konkreten Fall als selektives Lösungsmittel eingesetzt wurden.

Sofern es die thermische Belastbarkeit des selektiven Lösungsmittels zulässt, wird die Temperatur im Sumpf der Extraktivdestillationskolonne vorteilhaft so hoch eingestellt, dass am Kopf der Extraktivdestillationskolonne noch mit Flusswasser kondensiert werden kann.

Ist die thermische Belastbarkeit des konkret eingesetzten selektiven Lösungsmittels jedoch bei der Temperatur, die notwendig wäre, um am Sumpf gereinigtes Lösungsmittel zu erhalten, nicht ausreichend, muss bei einer Temperatur am Kolonnensumpf gearbeitet werden, die für das selektive Lösungsmittel noch zulässig ist und entsprechend am Kolonnenkopf mit einem aufwändigeren Kühlmittel gegenüber Flusswasser gekühlt werden.

Ein besonders bevorzugtes selektives Lösungsmittel ist, wie vorstehend ausgeführt, NMP, bevorzugt in wässriger Lösung, insbesondere mit 7 bis 10 Gew.-% Wasser, besonders bevorzugt mit 8,3 Gew.-% Wasser.

Unter der Voraussetzung, dass NMP als selektives Lösungsmittel eingesetzt wird, wird die Temperatur im Sumpf der Extraktivdestillationskolonne bevorzugt im Bereich zwischen 170 und 190, besonders bevorzugt bei 180°C eingestellt. Entsprechend wird der Kopfdruck im zweiten Teilbereich der als Trennwandkolonne ausgestalteten Extraktivdestillationskolonne im Bereich von 1 bis 10 bar absolut, bevorzugt von 2 bis 5 bar absolut, besonders bevorzugt bei 3,5 bar absolut, eingestellt.

Es ist grundsätzlich nicht erforderlich, die Gewinnung des Nebenprodukts aus Butanen und Butenen, des sogenannten Raffinats 1, in einer von der Extraktivdestillationskolonne getrennten, vorgeschalteten Extraktivwaschkolonne vorzusehen. Es ist auch möglich, die Extraktivwaschkolonne in den ersten Teilbereich der als Extraktivdestillationskolonne eingesetzten Trennwandkolonne zu integrieren, sofern es technisch und wirtschaftlich unter Berücksichtigung der konkreten Rahmenbedingungen für das Verfahren, insbesondere der Zusammensetzung des aufzutrennenden C₄-Schnitts und der Spezifikation für Raffinat 1 realisierbar ist, die Zahl der theoretischen Trennstufen im ersten Teilbereich der Trennwandkolonne entsprechend zu erhöhen.

Die nachstehend beschriebenen bevorzugten Verfahrensvarianten aus dem Verfahren der DE-A 101 05 660.5 sind auch für das Verfahren der vorliegenden Erfindung gleichermaßen anwendbar:

In einer bevorzugten Verfahrensvariante wird der Brüdenstrom am unteren Ende der Trennwand der Trennwandkolonne durch geeignete Maßnahmen dergestalt aufgetrennt, daß der Teilstrom, der in den ersten Teilbereich der Trennwandkolonne geleitet wird, größer ist als der Teilstrom, der in den zweiten Teilbereich der Trennwandkolonne geleitet wird. Durch die Regelung der Aufteilung des Brüdenstroms am unteren Ende der Trennwand kann in einfacher und zuverlässiger Weise die geforderte Produktspezifikation des am Kopf des zweiten Teilbereichs der Trennwandkolonne abgezogenen Roh-1,3-Butadien-Stroms gewährleistet werden.

Besonders bevorzugt kann eine derartige ungleiche Auftrennung des Brüdenstroms am unteren Ende der Trennwand dadurch erreicht werden, daß die Trennwand außermittig angeordnet wird, und zwar der Gestalt, daß der zweite Teilbereich kleiner ist gegenüber dem ersten Teilbereich der Trennwandkolonne.

Besonders bevorzugt wird die Trennwand außermittig der Gestalt angeordnet, daß das Querschnittsverhältnis des ersten Teilbereichs zum zweiten Teilbereich im Bereich von 8:1 bis 1,5:1, insbesondere bei 2,3:1 liegt.

Alternativ oder zusätzlich zur außermittigen Anordnung der Trennwand kann der Brüdenstrom am unteren Ende der Trennwand durch weitere Maßnahmen, beispielsweise Klappen oder Leitbleche im gewünschten Verhältnis auf die beiden Teilbereiche der Trennwandkolonne aufgeteilt werden.

Eine weitere zusätzliche oder alternative Maßnahme zur Aufteilung des Brüdenstroms am unteren Ende der Trennwand besteht in der Einstellung der Wärmeabführungsleistung des Kondensators am Kopf des zweiten Teilbereichs der Trennwandkolonne.

Eine bevorzugte Verfahrensvariante ist dadurch gekennzeichnet, daß die Drücke am oberen Ende der beiden Teilbereiche der Trennwandkolonne jeweils getrennt regelbar sind. Dadurch kann die erforderliche Produktspezifikation des Roh-1,3-Butadiens sichergestellt werden.

Die Kopfdrücke der beiden Teilbereiche der Trennwandkolonne können bevorzugt jeweils über eine Split-Range-Regelung eingestellt werden. Der Begriff Split-Range-Regelung bezeichnet in bekannter Weise eine Anordnung, wonach der Druckreglerausgang gleichzeitig mit der Inertgas- und der Entlüftungsleitung verbunden ist. Der Ventilstellbereich des Druckreglers ist so geteilt, daß jeweils nur ein Ventil betätigt wird, d.h. entweder es strömt Inertgas zu oder es wird entlüftet. Dadurch können die Inertgasmenge wie auch die mit dem Abluftstrom verbundenen Produktverluste minimiert werden.

Zusätzlich oder alternativ zur Split-Range-Regelung ist es möglich, die Kopfdrücke in den beiden Teilbereichen der Trennwandkolonne jeweils über die Wärmeabführleistung der Kondensatoren am Kopf des zweiten Teilbereichs der Trennwandkolonne bzw. am Kopf der Extraktivwaschkolonne zu regeln.

In einer bevorzugten Verfahrensvariante wird der Kopfdruck im zweiten Teilbereich der Trennwandkolonne größer als im ersten Bereich der Trennwandkolonne eingestellt, insbesondere um 1 - 100 mbar, besonders bevorzugt um 1 - 30 mbar. Durch diese Maßnahme ist es möglich, auf eine fest eingeschweißt oder aufwändig abgedichtete Trennwand zu verzichten und eine kostengünstigere lose Trennwand einzusetzen. Durch das vom zweiten zum ersten Teilbereich der Trennwandkolonne hin gerichtete Druckgefälle können flüssige oder gasförmige Leckargeströme nur in dieser Richtung auftreten, und sind somit unkritisch für die Reinheit des am Kopf des zweiten Teilbereichs abgezogenen Wertprodukts Roh-1,3-Butadien.

Der Wärmeinhalt des Sumpfstromes des gereinigten Lösungsmittels kann vorteilhaft durch Wärmeintegration im Verfahren selbst genutzt werden, insbesondere, in dem man von einer oder mehreren Stellen der Trennwandkolonne Flüssigkeit oder einen Teilstrom der Flüssigkeit aus dem unteren gemeinsamen Kolonnenbereich der Trennwandkolonne abzieht, durch indirekten Wärmetausch mit dem heißen Sumpfstrom aus der Extraktivdestillationskolonne erwärmt und/oder verdampft und erneut in den unteren gemeinsamen Kolonnenbereich zurückführt.

Bevorzugt wählt man hierbei die Trennstufe, von der man die Flüssigkeit oder den Teilstrom der Flüssigkeit abzieht, in der Weise, dass der Gesamtenergiebedarf für die Extraktivdestillationskolonne minimal ist.

Zusätzlich oder alternativ kann der Wärmeinhalt des Sumpfstroms des gereinigten Lösungsmittels auch zum indirekten Wärmetausch mit dem der Extraktivdestillationskolonne zuzuführenden C₄-Schnitt genutzt werden.

Bevorzugt wird aus dem unteren gemeinsamen Kolonnenbereich der Trennwandkolonne zwecks Ausschleusung der C₄-Acetylene aus dem Verfahren, insbesondere Ethylacetylen und Vinylacetylen ein Seitenstrom abgezogen, einer Waschkolonne zugeführt, in der der Seitenstrom mit Wasser gewaschen wird, aus der Waschkolonne ein Kopfstrom abgezogen, der teilweise oder vollständig, bevorzugt teilweise kondensiert wird, das Kondensat teilweise ausgeschleust und im übrigen als Rücklauf auf die Waschkolonne aufgegeben und der Sumpfstrom aus der Waschkolonne abgezogen und dem unteren gemeinsamen Kolonnenbereich erneut zugeführt.

Durch die Erfindung wird somit ein Verfahren zur Gewinnung von Roh-1,3-Butadien durch Extraktivdestillation aus einem C₄-Schnitt zur Verfügung gestellt, das gegenüber bekannten Verfahren die Rückgewinnung von gereinigtem Lösungsmittel, das vorzugsweise in das Verfahren recycliert wird, unmittelbar aus dem Sumpf der Extraktivdestillationskolonne ermöglicht. Durch die Einsparung der hierfür bislang erforderlichen Ausgaserkolonne und der zugehörigen Peripherie, insbesondere Wärmetauscher und Pumpen, besonders jedoch des Kompressors zur Verdichtung des in die Extraktivdestillationskolonne zurückzuführenden Kohlenwasserstoffstromes sind die Investitionskosten gegenüber bekannten Verfahren niedriger. Besonders vorteilhaft ist der Wegfall des Kompressors, der im Verfahren nach dem Stand der Technik der bei weitem größte Stromverbraucher ist. Durch den Wegfall des Kompressors sinkt der Verbrauch an elektrischer Energie im erfindungsgemäßen Verfahren gegenüber dem Verfahren nach dem Stand der Technik um etwa die Hälfte.

Die Erfindung wird im folgenden anhand einer Zeichnung näher erläutert:

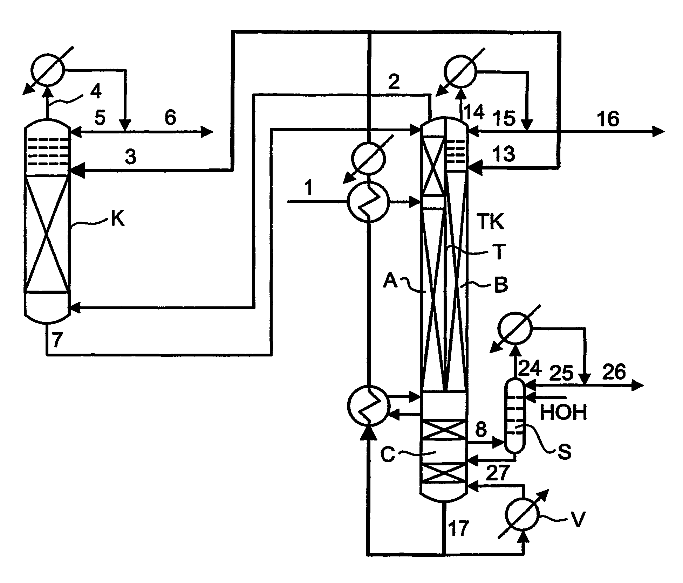

- Figur 1: zeigt das Schema einer Anlage nach der Erfindung

In einer Trennwandkolonne TK mit einer in Kolonnenlängsrichtung angeordneten Trennwand T, die die Trennwandkolonne in einen ersten Teilbereich A, einen zweiten Teilbereich B und einen unteren gemeinsamen Kolonnenbereich C aufteilt, wird dem ersten Teilbereich A ein C₄-Schnitt 1 zugeführt. Beispielhaft enthält der zweite Teilbereich B 40 theoretische Trennstufen und der untere gemeinsame Kolonnenbereich C 10 theoretische Trennstufen. Der Kopfstrom 2 aus dem Teilbereich A wird in den unteren Bereich der vorgeschalteten Extraktivwaschkolonne K mit beispielhaft 19 theoretischen Trennstufen geleitet. Die Extraktivwaschkolonne K wird mit einem ersten Lösungsmittelteilstrom 3, in deren oberen Bereich, beaufschlagt, es findet eine Gegenstromextraktion statt, wobei ein Sumpfstrom 7 anfällt, der zurück in den oberen Bereich des Teilbereichs A der Trennwandkolonne TK geführt wird und ein Kopfstrom 4, der in einem Kondensator am Kopf der Extraktivwaschkolonne K kondensiert wird, wobei ein Teilstrom des Kondensats als Strom 5 erneut auf die Extraktivwaschkolonne K aufgegeben und das Kondensat im übrigen als Strom 6 abgezogen wird.

Die Trennwandkolonne TK wird in deren zweiten Teilbereich B mit einem zweiten Lösungsmittelteilstrom 13 beaufschlagt. Aus dem zweiten Teilbereich B wird ein Kopfstrom 14 abgezogen und kondensiert, ein Teilstrom 15 des kondensierten Kopfstroms 14 als Rücklauf auf den zweiten Teilbereich B der Trennwandkolonne gegeben und der kondensierte Kopfstrom 14 im übrigen als Roh-1,3-Butadien (Strom 16) abgezogen.

Aus dem unteren gemeinsamen Kolonnenbereich C wird der in der Figur dargestellten bevorzugten Ausführungsform ein Seitenstrom 8 abgezogen, einer Waschkolonne S zugeführt und in der Waschkolonne S mit Wasser (Strom HOH) gewaschen. Aus der Waschkolonne S wird ein Kopfstrom 24 abgezogen, in einem Kondensator kondensiert, ein Teil des Kondensats (Strom 25) als Rücklauf erneut auf die Waschkolonne S aufgegeben und im übrigen als Strom 26, enthaltend die C₄-Acetylene, ausgeschleust. Aus dem Sumpf der Waschkolonne S wird ein Strom 27 abgezogen und erneut dem unteren gemeinsamen Kolonnenbereich C der Trennwandkolonne TK zugeführt. Der Sumpfverdampfer der Trennwandkolonne TK ist mit V bezeichnet. Der Sumpfstrom 17 der Trennwandkolonne TK, der aus gereinigtem Lösungsmittel, beispielhaft NMP mit 8,3 Gew.-% Wasser besteht, wird zur Wärmeintegration mit einem Flüssigkeitsstrom genutzt, der aus dem unteren gemeinsamen Kolonnenbereich C der Trennwandkolonne TK abgezogen, und, nach Aufheizung durch den Strom 17, erneut in den unteren gemeinsamen Kolonnenbereich zugeführt wird. Darüber hinaus kann, wie in der Figur beispielhaft dargestellt, der Wärmeinhalt des Sumpfstroms 17 vorteilhaft zur Vorerwärmung des der Trennwandkolonne TK zugeführten C₄-Schnittes (Strom 1) genutzt werden.

## Patentansprüche

1. Verfahren zur Gewinnung von Roh-1,3-Butadien durch Extraktivdestillation aus einem C₄-Schnitt mit einem selektiven Lösungsmittel in einer Trennwandkolonne (TK), in der eine Trennwand (T) in Kolonnenlängsrichtung unter Ausbildung eines ersten Teilbereichs (A), eines zweiten Teilbereichs (B) und eines unteren gemeinsamen Kolonnenbereichs (C) angeordnet und der eine Extraktivwaschkolonne (K) vorgeschaltet ist, **dadurch gekennzeichnet, dass** man durch Regelung des Energieeintrags in die Trennwandkolonne (TK) über einen Sumpfverdampfer (V) und Auslegung der Zahl der theoretischen Trennstufen im unteren gemeinsamen Kolonnenbereich (C) die Fahrweise der Trennwandkolonne (TK) so einstellt, dass man aus der Trennwandkolonne (TK) einen Sumpfstrom (17) erhält, der aus gereinigtem Lösungsmittel besteht.

2. Verfahren nach Anspruch 1, **dadurch gekennzeichnet, dass** man die Flüssigkeit oder einen Teilstrom der Flüssigkeit von einer oder mehreren Stellen aus dem unteren gemeinsamen Kolonnenbereich (C) abzieht, durch indirekten Wärmetausch mit dem Sumpfstrom (17) aus der Trennwandkolonne (TK) erwärmt und/oder verdampft und erneut dem unteren gemeinsamen Kolonnenbereich (C) der Trennwandkolonne (TK) zurückführt.

3. Verfahren nach Anspruch 2, **dadurch gekennzeichnet, dass** man die Flüssigkeit oder den Teilstrom der Flüssigkeit aus dem unteren gemeinsamen Kolonnenbereich (C) der Trennwandkolonne (TK) von einer Trennstufe abzieht, die man so wählt, dass der Energiebedarf für die Trennwandkolonne (TK) minimal ist.

4. Verfahren nach einem der Ansprüche 1 bis 3, **dadurch gekennzeichnet, dass** man einen Teil der Energie des Sumpfstroms (17) aus der Trennwandkolonne (TK) zum indirekten Wärmetausch mit dem der Trennwandkolonne (TK) zugeführten aufzutrennenden C4-Schnitt (19) nutzt.

5. Verfahren nach einem der Ansprüche 1 bis 4, **dadurch gekennzeichnet, dass** man die Extraktivwaschkolonne (K) konstruktiv in den ersten Teilbereich (A) der Trennwandkolonne (TK) integriert, indem man die Zahl der theoretischen Trennstufen im ersten Teilbereich (A) der Trennwandkolonne (TK) entsprechend größer auslegt.

6. Verfahren nach einem der Ansprüche 1 bis 5, **dadurch gekennzeichnet, dass** man aus dem unteren gemeinsamen Kolonnenbereich (C) der Trennwandkolonne (TK) einen Seitenstrom (8) abzieht, den Seitenstrom (8) einer Waschkolonne (S) zuführt, in der man eine Wasserwäsche durchführt, aus der Waschkolonne (S) einen Kopfstrom (24) abzieht, in einem Kondensator am Kolonnenkopf vollständig oder bevorzugt teilweise kondensiert, teilweise als Rücklauf (25) wieder auf die Waschkolonne (S) aufgibt und im übrigen als Strom (26) ausschleust und den Sumpfstrom (27) aus der Waschkolonne (S) in den unteren gemeinsamen Kolonnenbereich (C) der Trennwandkolonne (TK) zurückführt.

7. Verfahren nach einem der Ansprüche 1 bis 6, **dadurch gekennzeichnet, dass**
- der C₄-Schnitt (1) dem ersten Teilbereich (A) der Trennwandkolonne (TK), bevorzugt in dessen mittleren Bereich, zugeführt wird,
- der Kopfstrom (2) aus dem ersten Teilbereich (A) der Trennwandkolonne (TK) der Extraktivwaschkolonne (K), in deren unteren Bereich, zugeführt wird,
- in der Extraktivwaschkolonne (K) eine Gegenstromextraktion durch Beaufschlagung mit einem ersten Teilstrom (3) des selektiven Lösungsmittels im oberen Bereich der Extraktivwaschkolonne (K) durchgeführt wird,
- die im selektiven Lösungsmittel weniger als 1,3-Butadien löslichen Komponenten des C₄-Schnittes über Kopf der Extraktivwaschkolonne (K) abgezogen (4) werden,
- der Sumpfstrom (7) aus der Extraktivwaschkolonne (K) in den oberen Bereich des ersten Teilbereichs (A) der Trennwandkolonne (TK) zurückgeführt wird,
- der Trennwandkolonne (TK) im oberen Bereich des zweiten Teilbereichs (B) ein zweiter Teilstrom (13) des selektiven Lösungsmittels zugeführt wird,
- das Kopfprodukt (14) aus dem zweiten Teilbereich (B) der Trennwandkolonne (TK) als Roh-1,3-Butadien (16) abgezogen wird und
- aus dem unteren gemeinsamen Kolonnenbereich (C) der Trennwandkolonne (TK) ein Sumpfstrom (17), bestehend aus gereinigtem Lösungsmittel, abgezogen wird, der in das Verfahren recycliert wird.

8. Verfahren nach einem der Ansprüche 1 bis 7, **dadurch gekennzeichnet, dass** man als selektives Lösungsmittel N-Methylpyrrolidon, bevorzugt in wässriger Lösung, insbesondere mit 7 bis 10 Gew.-% Wasser, besonders bevorzugt mit 8,3 Gew.-% Wasser einsetzt.

9. Verfahren nach einem der Ansprüche 1 bis 8, **dadurch gekennzeichnet, dass** man die Temperatur im Sumpf der Trennwandkolonne (TK) im Bereich von 170 bis 190°C, bevorzugt auf 180°C und den Druck am Kopf des zweiten Teilbereichs (B) der Trennwandkolonne (TK) im Bereich von 1 bis 10 bar absolut, bevorzugt von 2 bis 5 bar absolut, besonders bevorzugt auf 3,5 bar absolut, regelt.

## Claims

1. A process for recovering crude 1,3-butadiene from a C₄ fraction by extractive distillation using a selective solvent in a dividing wall column (TK) in which a dividing wall (T) is arranged in the longitudinal direction of the column to form a first subregion (A), a second subregion (B) and a lower common column region (C) and which is preceded by an extractive scrubbing column (K), wherein the operation of the dividing wall column (TK) is set by regulation of the energy input into the dividing wall column (TK) via a bottom vaporizer (V) and setting of the number of the theoretical plates in the lower common column region (C) so that a bottom stream (17) consisting of purified solvent is obtained from the dividing wall column (TK).

2. The process according to claim 1, wherein the liquid or a substream of the liquid is taken off from the lower common column region (C) at one or more points, heated and/or vaporized by indirect heat exchange with the bottom stream (17) from the dividing wall column (TK) and fed back into the lower common column region (C) of the dividing wall column (TK).

3. The process according to claim 2, wherein the liquid or the substream of the liquid is taken off from the lower common column region (C) of the dividing wall column (TK) at a theoretical plate which is selected so that the energy requirement for the dividing wall column (TK) is minimized.

4. The process according to any of claims 1 to 3, wherein part of the energy of the bottom stream (17) from the dividing wall column (TK) is utilized for indirect heat exchange with the C4 fraction (19) to be fractionated which is fed to the dividing wall column (TK).

5. The process according to any of claims 1 to 4, wherein the extractive scrubbing column (K) is structurally integrated into the first subregion (A) of the dividing wall column (TK) by making the number of theoretical plates in the first subregion (A) of the dividing wall column (TK) correspondingly larger.

6. The process according to any of claims 1 to 5, wherein a side stream (8) is taken off from the lower common column region (C) of the dividing wall column (TK), the side stream (8) is fed to a scrubbing column (S) in which a water scrub is carried out, a top stream (24) is taken off from the scrubbing column (S), condensed completely or preferably partially in a condenser at the top of the column, part of the condensate is returned as runback (25) to the scrubbing column (S) and the remainder is discharged as stream (26) and the bottom stream (27) from the scrubbing column (S) is recirculated to the lower common column region (C) of the dividing wall column (TK).

7. The process according to any of claims 1 to 6, wherein
- the C₄ fraction (1) is fed to the first subregion (A) of the dividing wall column (TK), preferably in its middle region,
- the stream (2) taken off at the top from the first subregion (A) of the dividing wall column (TK) is fed to the extractive scrubbing column (K) in its upper region,
- a countercurrent extraction is carried out in the extractive scrubbing column (K) by treatment with a first substream (3) of the selective solvent in the upper region of the extractive scrubbing column (K),
- the components of the C₄ fraction which are less soluble than 1,3-butadiene in the selective solvent are taken off (4) at the top of the extractive scrubbing column (K),
- the bottom stream (7) from the extractive scrubbing column (K) is recirculated to the upper region of the first subregion (A) of the dividing wall column (TK),
- a second substream (13) of the selective solvent is fed to the dividing wall column (TK) in the upper region of the second subregion (B),
- the top product (14) from the second subregion (B) of the dividing wall column (TK) is taken off as crude 1,3-butadiene (16) and
- a bottom stream (17) consisting of purified solvent is taken off from the lower common column region (C) of the dividing wall column (TK) and is recycled to the process.

8. The process according to any of claims 1 to 7, wherein the selective solvent used is N-methylpyrrolidone, preferably in aqueous solution, in particular with a water content of from 7 to 10% by weight, particularly preferably with a water content of 8.3% by weight.

9. The process according to any of claims 1 to 8, wherein the temperature in the bottom of the dividing wall column (TK) is regulated in the range from 170 to 190°C, preferably at 180°C, and the pressure at the top of the second subregion (B) of the dividing wall column (TK) is regulated in the range from 1 to 10 bar absolute, preferably from 2 to 5 bar absolute, particularly preferably at 3.5 bar absolute.

## Revendications

1. Procédé de production de 1,3-butadiène brut par distillation extractive à partir d'une fraction en C₄ avec un solvant sélectif dans une colonne à paroi séparatrice (TK), dans laquelle une paroi séparatrice (T) est agencée dans la direction longitudinale de la colonne de manière à former une première zone partielle (A), une deuxième zone partielle (B) et une zone de colonne commune inférieure (C) et en amont de laquelle est montée une colonne de lavage extractif (K), **caractérisé en ce que** l'on ajuste le mode de fonctionnement de la colonne à paroi séparatrice (TK) en régulant l'apport d'énergie dans la colonne à paroi séparatrice (TK) via un évaporateur de bas de colonne (V) et en dimensionnant le nombre des étages théoriques dans la zone de colonne commune inférieure (C) de manière à obtenir de la colonne à paroi séparatrice (TK) un courant de bas de colonne (17) qui est constitué de solvant purifié.

2. Procédé selon la revendication 1, **caractérisé en ce que** l'on soutire le liquide ou un courant partiel du liquide d'un ou plusieurs points de la zone de colonne commune inférieure (C), on le chauffe et/ou on l'évapore par échange de chaleur indirect avec le courant de bas de colonne (17) de la colonne à paroi séparatrice (TK) et on le renvoie à nouveau à la zone de colonne commune inférieure (C) de la colonne à paroi séparatrice (TK).

3. Procédé selon la revendication 2, **caractérisé en ce que** l'on soutire le liquide ou le courant partiel du liquide de la zone de colonne commune inférieure (C) de la colonne à paroi séparatrice (TK) d'un étage de séparation que l'on choisit de sorte que le besoin en énergie pour la colonne à paroi séparatrice (TK) soit minimal.

4. Procédé selon l'une quelconque des revendications 1 à 3, **caractérisé en ce que** l'on utilise une partie de l'énergie du courant de bas de colonne (17) de la colonne à paroi séparatrice (TK) pour l'échange de chaleur indirect avec la fraction en C₄ (19) à séparer qui est acheminée à la colonne à paroi séparatrice (TK).

5. Procédé selon l'une quelconque des revendications 1 à 4, **caractérisé en ce que** l'on intègre la colonne de lavage extractif (K) structurellement à la première zone partielle (A) de la colonne à paroi séparatrice (TK) en augmentant de manière correspondante le nombre des étages de séparation théoriques dans la première zone partielle (A) de la colonne à paroi séparatrice (TK).

6. Procédé selon l'une quelconque des revendications 1 à 5, **caractérisé en ce que** l'on soutire de la zone de colonne commune inférieure (C) de la colonne à paroi séparatrice (TK) un courant latéral (8), **en ce que** l'on achemine le courant latéral (8) à une colonne de lavage (S), dans laquelle on effectue une lessive à l'eau, **en ce que** l'on soutire de la colonne de lavage (S) un courant de tête (24), **en ce qu'**on le condense dans un condenseur en tête de colonne complètement ou de préférence partiellement, **en ce qu'**on le délivre partiellement comme reflux (25) de nouveau à la colonne de lavage (S), **en ce qu'**on l'évacue par ailleurs par éclusage sous la forme d'un courant (26) et **en ce qu'**on renvoie le courant de bas de colonne (27) de la colonne de lavage (S) dans la zone de colonne commune inférieure (C) de la colonne à paroi séparatrice (TK).

7. Procédé selon l'une quelconque des revendications 1 à 6, **caractérisé en ce que** :
- l'on achemine la fraction en C₄ (1) à la première zone partielle (A) de la colonne à paroi séparatrice (TK), de préférence dans sa zone centrale,
- l'on achemine le courant de tête (2) de la première zone partielle (A) de la colonne à paroi séparatrice (TK) à la colonne de lavage extractif (K), dans sa zone inférieure,
- l'on effectue dans la colonne de lavage extractif (K) une extraction à contre-courant par injection d'un premier courant partiel (3) du solvant sélectif dans la zone supérieure de la colonne de lavage extractif (K),
- l'on soutire (4) les composants de la fraction en C₄ moins solubles que le 1,3-butadiène dans le solvant sélectif par la tête de la colonne de lavage extractif (K),
- l'on renvoie le courant de bas de colonne (7) dé la colonne de lavage extractif (K) dans la zone supérieure de la première zone partielle (A) de la colonne à paroi séparatrice (TK),
- l'on achemine à la colonne à paroi séparatrice (TK) dans la zone supérieure de la deuxième zone partielle (B) un deuxième courant partiel (13) du solvant sélectif,
- l'on soutire le produit en tête (14) de la deuxième zone partielle (B) de la colonne à paroi séparatrice (TK) sous forme de 1,3-butadiène brut (16), et
- l'on soutire de la zone de colonne commune inférieure (C) de la colonne à paroi séparatrice (TK) un courant de bas de colonne (17), constitué d'un solvant purifié qui est recyclé dans le procédé.

8. Procédé selon l'une quelconque des revendications 1 à 7, **caractérisé en ce que** l'on utilise de la N-méthylpyrrolidone comme solvant sélectif, de préférence en solution aqueuse, en particulier avec 7 à 10 % en poids d'eau, mieux encore avec 8,3 % en poids d'eau.

9. Procédé selon l'une quelconque des revendications 1 à 8, **caractérisé en ce que** l'on règle la température dans le bas de la colonne à paroi séparatrice (TK) dans la plage de 170 à 190 °C, de préférence à 180 °C, et la pression en tête de la deuxième zone partielle (B) de la colonne à paroi séparatrice (TK) dans la plage de pression absolue de 1 à 10 bars, de préférence de 2 à 5 bars, mieux encore de 3,5 bars.
